**Europäisches Patentamt**

**European Patent Office**

**Office européen des brevets**

(19)

(11) Numéro de publication : **0 451 016 B1**

(12) **FASCICULE DE BREVET EUROPEEN**

(45) Date de publication du fascicule du brevet :
18.05.94 Bulletin 94/20

(51) Int. Cl.⁵ : **C07C 19/08,** C09K 5/00,
C09K 3/30, C08J 9/14

(21) Numéro de dépôt : **91400803.2**

(22) Date de dépôt : **26.03.91**

(54) **Nouveau mélange azéotropique à bas point d'ébullition et ses applications comme fluide frigorigène, comme propulseur d'aérosols ou comme agent d'expansion des mousses plastiques.**

(30) Priorité : **02.04.90 FR 9004168**

(43) Date de publication de la demande :
**09.10.91 Bulletin 91/41**

(45) Mention de la délivrance du brevet :
**18.05.94 Bulletin 94/20**

(84) Etats contractants désignés :
**BE CH DE ES FR GB IT LI NL**

(56) Documents cités :
**EP-A- 0 325 265**
**US-A- 4 842 764**
**RESEARCH DISCLOSURE, octobre 1977, page 70, disclosure no. 16265, Havant, Hampshire, GB; "Fluorocarbon azeotropes"**

(73) Titulaire : **ELF ATOCHEM S.A.**
**4 & 8, Cours Michelet La Défense 10**
**F-92800 Puteaux (FR)**

(72) Inventeur : **Arnaud, Didier**
**12 Rue Camille Saint-Saens**
**F-92400 Courbevoie (FR)**
Inventeur : **Tanguy, Jean-Claude**
**7 Rue du Lieutenant Keiser**
**F-95110 Sannois (FR)**

(74) Mandataire : **Leboulenger, Jean et al**
**ATOCHEM Département Propriété Industrielle**
**F-92091 Paris la Défense 10 Cédex 42 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un mélange de fluides frigorigènes à très bas point d'ébullition, n'ayant pas d'action sur l'ozone stratosphérique et utilisable pour remplacer le trifluorobromométhane dans les systèmes de réfrigération industrielle très basse température à compression monoétagée.

Il est maintenant établi qu'à cause de son coefficient important d'action sur l'ozone, le trifluorobromométhane (Halon 1301) devra, à plus ou moins longue échéance, être remplacé par des fluides frigorigènes ne contenant plus de chlore ni de brome et, de ce fait, n'ayant pas d'action sur l'ozone stratosphérique.

Compte tenu de sa très faible action sur l'environnement, le 1,1,1-trifluorcéthane (HFA 143a) a déjà été proposé comme substitut aux CFC (chlorofluorocarbures). Cependant, en raison de son point d'ébullition (-47,6°C), l'utilisation du HFA 143a seul est réservée aux applications à températures d'évaporation voisines de -45°C et ne peut pas être envisagée pour les applications à très basses températures d'ébullition (-60°C à -50°C par exemple). Il en est de même pour l'azéotrope 1,1,1,2-tétrafluoroéthane/propane qui est mentionné dans le document Research Disclosure, Octobre 1977, page 70, n° 16265 et dont le point d'ébullition se situe entre -42 et -45°C.

En effet, la température minimale atteinte à l'évaporateur est, dans la pratique, limitée par la valeur de la température d'ébullition normale du fluide frigorigène afin d'éviter l'introduction d'air ou de saumure dans l'installation en cas de fuites à l'évaporateur, ce qui risquerait de perturber le fonctionnement normal du système.

Il a maintenant été trouvé que le 1,1,1-trifluoroéthane (HFA 143a) et le propane (R 290) forment un azéotrope à point d'ébullition minimum égal à environ - 53,4°C à 1,013 bar et dont la teneur en R 290 au point d'ébullition normal est d'environ 29,4 % en masse. Bien entendu, cette composition varie en fonction de la pression du mélange et, à une pression donnée, peut être facilement déterminée suivant des techniques bien connues.

Du fait de son très bas point d'ébullition, le mélange azéotropique selon l'invention peut être utilisé comme fluide frigorigène dans les applications industrielles aux basses températures d'ébullition (-50°C) monoétagées comme dans le cas des tunnels de surgélation, où le R 290 et le HFA 143a purs ne peuvent pas être utilisés compte tenu de la valeur trop élevée de leur point d'ébullition.

Etant donné ses propriétés physiques proches de celles des CFC, le mélange selon l'invention peut également être utilisé comme propulseur d'aérosols ou comme agent d'expansion des mousses plastiques.

Les exemples suivants illustrent l'invention, sans la limiter.

## EXEMPLE 1

L'azéotrope selon l'invention a été étudié expérimentalement à différentes températures par analyse, en chromatographie phase gaz, des compositions de la phase liquide et de la phase vapeur pour différents mélanges de HFA 143a et R 290.

Les pressions ont été mesurées avec une précision supérieure à 0,02 bar au moyen d'un manomètre HEISE. Les températures ont été mesurées à 0,02°C près au moyen d'une sonde de platine 1 000 ohms.

Le Graphe 1 en annexe représente la courbe d'équilibre liquide/vapeur des mélanges HFA143a/R290, établie à la température de 20,1°C. Sur ce graphe, l'axe des abcisses indique la fraction massique en HFA 143a et l'axe des ordonnées la pression absolue en bars ; les carrés noirs correspondent aux points expérimentaux.

Pour chaque température, on obtient une courbe analogue à celle du Graphe 1. Par ajouts successifs de HFA 143a dans le R 290, la pression développée par le mélange augmente régulièrement, puis passe par un maximum et décroît régulièrement ce qui met en évidence l'existence de l'azéotrope à point d'ébullition minimum.

La corrélation des points expérimentaux ainsi obtenus pour plusieurs isothermes a été effectuée selon des techniques bien connues, au moyen d'une simulation informatique.

Les points normaux d'ébullition ainsi déterminés pour différentes compositions en HFA 143a, sont résumés dans le tableau 1 suivant :

TABLEAU 1

| Composition massique en HFA 143a % | Point d'ébullition normal °C |
|---|---|
| 100 | − 46,7 |
| 90 | − 52,3 |
| 80 | − 53,3 |
| 70,6 | − 53,4 |
| 70 | − 53,3 |
| 60 | − 53,2 |
| 50 | − 53,1 |
| 40 | − 52,7 |
| 30 | − 51,7 |
| 20 | − 49,9 |
| 10 | − 46,7 |
| 0 | − 41,5 |

Les résultats de ces corrélations mettent en évidence le minimum du point d'ébullition normal pour une fraction massique du HFA 143a égale à 70,6 % ; ce qui permet de caractériser l'azéotrope par :
. son point d'ébullition normal qui est égal à environ -53,4°C
. sa composition massique en HFA 143a égale à environ 70,6 %
Le tableau 2 suivant donne la relation pression/température pour cet azéotrope, comparée à celle des corps purs

TABLEAU 2

| Température (°C) | Pression absolue (bar) | | |
|---|---|---|---|
| | Azéotrope HFA 143a/R 290 | HFA 143a pur | R 290 pur |
| − 50,0 | 1,19 | 0,90 | 0,68 |
| − 20,0 | 3,93 | 3,22 | 2,43 |
| − 0,2 | 7,40 | 6,19 | 4,71 |
| + 20,1 | 12,9 | 11, 2 | 8,41 |
| + 50,0 | 25,8 | 23,1 | 17,0 |

La tension de vapeur de l'azéotrope reste sur une large gamme de température supérieure à la tension de vapeur des corps purs ; ces données indiquent que le mélange reste azéotropique dans tout cet intervalle de température.

## EXEMPLE 2

Cet exemple illustre l'utilisation de l'azéotrope selon l'invention comme fluide frigorigène.
Les performances thermodynamiques du mélange azéotropique selon l'invention ont été comparées aux performances des deux constituants seuls, dans des conditions proches de celles rencontrées dans les tunnels de surgélation monoétagés à savoir les suivantes :
. température de condensation : + 30°C

. température d'évaporation : - 50°C
. sous-refroidissement liquide : + 5°C
. surchauffe des vapeurs à l'aspiration du compresseur : + 20°C

Le tableau 3 résume les performances thermodynamiques observées dans ces conditions pour le HFA 143a pur, le R 290 pur et le mélange azéotropique selon l'invention.

### TABLEAU 3

|  | Azéotrope HFA 143a/R 290 | HFA 143a pur | R 290 pur |
|---|---|---|---|
| Pression évaporation (bar) | 1,19 | 0,90 | 0,68 |
| Pression condensation (bar) | 16,5 | 14,4 | 10,9 |
| Température de refoulement (°C) | 64,1 | 68,4 | 69,3 |
| Taux de compression | 13,9 | 16,0 | 15,9 |
| Puissance frigorifique ($kJ/m^3$) | 623,6 | 527,1 | 428,5 |
| Coefficient de performance | 1,78 | 1,86 | 1,97 |

On peut observer que le mélange azéotropique selon l'invention offre un certain nombre d'avantages sur le HFA 143a pur ou le R 290 pur, notamment :

. une température de refoulement plus faible assurant une durée de vie plus longue du compresseur.

. un taux de compression plus faible, donc une amélioration du rendement volumétrique du compresseur et par conséquent des coûts moindres d'exploitation de l'installation.

. une puissance frigorifique volumétrique disponible considérablement plus élevée, ce qui pratiquement, pour une puissance frigorifique donnée, permet l'utilisation d'un compresseur plus petit que celui défini pour utiliser le HFA 143a pur ou le R 290 pur.

Cet accroissement de puissance frigorifique volumétrique disponible, dans le cas de l'azéotrope selon l'invention, permet également d'accroître de 18 % la puissance frigorifique disponible d'une installation déjà existante définie au HFA 143a.

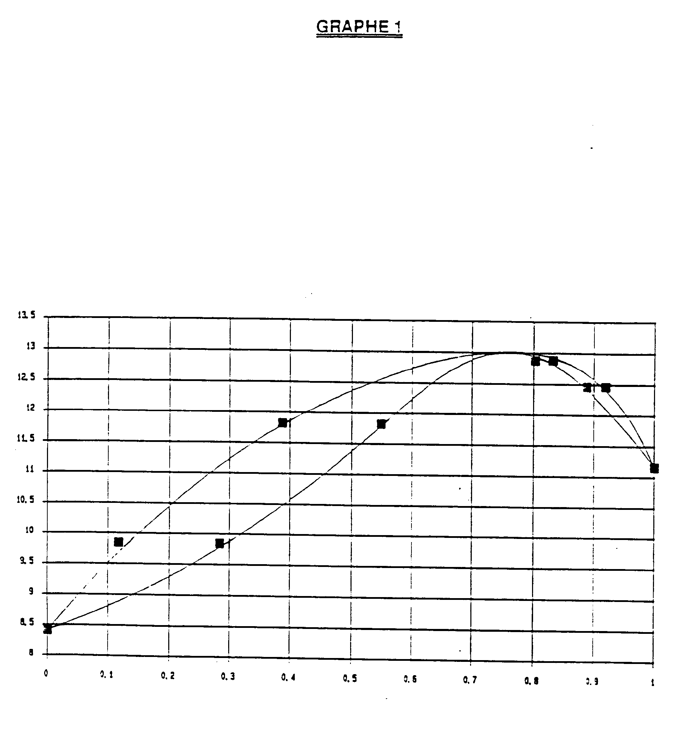

## Revendications

1. Azéotrope à point d'ébullition minimum, caractérisé en ce qu'il consiste d'un mélange de 1,1,1-trifluoroéthane et de propane et qu'à son point d'ébulition normal (environ -53,4°C sous 1,013 bar) il contient environ 70,6 % en masse de 1,1,1-trifluoroéthane et 29,4 % en masse de propane.

2. Utilisation de l'azéotrope selon la revendication 1 comme fluide frigorigène.

3. Utilisation de l'azéotrope selon la revendication 1 comme propulseur d'aérosols.

4. Utilisation de l'azéotrope selon la revendication 1 comme agent d'expansion des mousses plastiques.

**Patentansprüche**

1. Azeotrop mit niedrigem Siedepunkt, dadurch gekennzeichnet, daß es ein Gemisch aus 1,1,1-Trifluorethan und Propan enthält und daß es an seinem normalen Siedepunkt (-53,4°C bei 1,013 bar) etwa 70,6 Masse-% 1,1,1-Trifluorethan und 29,4 Masse-% Propan enthält.

2. Verwendung des Azeotrops nach Anspruch 1 als Kältemittel.

3. Verwendung des Azeotrops nach Anspruch 1 als Treibmittel.

4. Verwendung des Azeotrops nach Anspruch 1 als Schwellmittel für Plastikschaum.


**Claims**

1. Azeotrope with a minimum boiling point, characterized in that it consists of a mixture of 1,1,1-trifluoroethane and propane and in that at its normal boiling point (approximately -53.4°C at 1.013 bar) it contains approximately 70.6 mass% of 1,1,1-trifluoroethane and 29.4 mass% of propane.

2. Use of the azeotrope according to Claim 1 as refrigerating fluid.

3. Use of the azeotrope according to Claim 1 as a propellant for aerosols.

4. Use of the azeotrope according to Claim 1 as blowing agent for plastic foams.

## GRAPHE 1